Europäisches Patentamt

European Patent Office

Office européen des brevets

(11) Publication number: **0 069 488**
**B1**

(12) **EUROPEAN PATENT SPECIFICATION**

(45) Date of publication of patent specification: **11.06.86**

(51) Int. Cl.⁴: **C 12 Q 1/58, C 12 Q 1/32**

(21) Application number: **82303157.0**

(22) Date of filing: **17.06.82**

(54) Method for the purification of urease and glutamate dehydrogenase.

(30) Priority: **08.07.81 GB 8121131**

(43) Date of publication of application:
**12.01.83 Bulletin 83/02**

(45) Publication of the grant of the patent:
**11.06.86 Bulletin 86/24**

(84) Designated Contracting States:
**BE CH DE FR GB IT LI NL SE**

(56) References cited:
**EP-A-0 035 831**
**US-A-3 527 674**
**US-A-3 989 594**

**NATURE, vol. 287, no. 5781, October 1980,
pages 396-401, Chesham, Bucks, (GB); J.D.
WINDSASS et al.: "Improved conversion of
methanol to single-cell protein by
Methylophilus methylotrophus".
BIOCHIMICA ET BIOPHYSICA ACTA, vol. 146,
1967, pages 205-218; K.R. LYNN: "Some
properties and purifications of urease"**

(73) Proprietor: **IMPERIAL CHEMICAL INDUSTRIES
PLC
Imperial Chemical House Millbank
London SW1P 3JF (GB)**

(72) Inventor: **Senior, Peter James
Foulis Cottage Ingleby Greenhow
Middlesbrough Cleveland (GB)**
Inventor: **Collins, Stephen Hugh
Belcroft Cottage Cowesby
Thirsk N Yorkshire (GB)**

(74) Representative: **Aufflick, James Neil et al
Imperial Chemical Industries PLC Legal
Department: Patents PO Box 6 Bessemer Road
Welwyn Garden City Herts, AL7 1HD (GB)**

Courier Press, Leamington Spa, England.

## Description

This invention relates to a method for the purification together of the enzymes urease and glutamate dehydrogenase (GDH) from the same microorganism source to produce the enzyme component of a urea assay mixture which can be used in a method of testing for urea in physiological fluids and particularly blood serum.

A test for urea in clinical blood samples which is widely used in hospital pathological laboratories relies upon the two enzymes glutamate dehydrogenase (GDH) and urease. The test is carried out using an assay mixture containing the two enzymes and cofactors NAD(P)H (i.e. the reduced form of nicotinamide adenine dinucleotide (phosphate)—the cofactor of the GDH enzyme) and α-ketoglutarate in a suitable buffered medium. In assay mixtures which are commercially available at the present time the two enzymes have been derived from different sources, i.e. urease from Jack bean meal and GDH from bovine liver, and have been separately purified from those sources.

According to the present invention we provide a method for the purification together of the enzymes urease and glutamate dehydrogenase (GDH) from the same micro-organism source which comprises a step (A) in which a solution containing the enzymes is contacted with ammonium sulphate under conditions such that a precipitate containing both enzymes is produced followed by a step (B) in which the precipitate is re-dissolved, the resulting solution is applied to an ion exchange material, suitably a dextran, and a liquid fraction containing the enzymes is collected.

The method of the invention provides a mixture of the enzymes urease and GDH which can form the enzyme component of a urea assay mixture which is useful for determining urea in physiological fluids and in particular for determining blood urea.

The reaction mechanism by which the assay mixture converts urea from e.g. blood into glutamate involves two reactions. In a first reaction, which is catalysed by the enzyme urease, urea is converted into carbon dioxide and ammonia. In a second reaction, which is catalysed by the GDH enzyme, the ammonia produced by the first reaction and α-ketoglutarate are converted into glutamate.

During this second reaction cofactor NAD(P)H is converted into NAD(P) (i.e. the cofactor in its oxidised form). This conversion of NAD(P)H to NAD(P) provides a means by which the extent of the reaction and hence the amount of urea in a blood sample can be determined by UV spectrophotometry. NAD(P)H absorbs at 340 nm in the UV whilst NAD(P) does not absorb at this wavelength. Hence the urea concentration in e.g. blood is determined from measurements of the decline in the height of the peak at 340 nm in the UV spectrum of a sample under test.

The source micro-organism for the enzyme component of the assay mixture may be any micro-organism which contains both enzymes and from which they may be purified. The micro-organism may be one which contains both enzymes naturally or it may be one into which DNA material comprising the gene or genes specifying either or both enzymes has been introduced by a genetic manipulation technique. Preferably the source micro-organism is a bacterium. Useful bacterial strains are urease-containing strains of the species *Methylophilus methylotrophus* (formerly named *Pseudomonas methylotropha*) which have been genetically modified to include in them the GDH enzyme. Cultures of *Methylophilus methylotrophus* strains which could be genetically modified in this way to make them suitable for use as source micro-organisms for the enzyme component of the present invention are described in our UK Patent No. 1370892 and are deposited as follows:—

NCIB: 10508—10515 and 10592—10596, all inclusive;
NRRL: B 5352—B 5364 inclusive;
FRI: 1215—1227 inclusive.

Especially useful are GDH-containing strains derived from one of the above-mentioned *Methylophilus methylotrophus* strains, strain NCIB 10515, by genetic manipulation, in particular the modified GDH-containing strain NCIB 11585 and variants and mutants thereof. Genetically modified GDH-containing strains of *Methylophilus methylotrophus*, in particular strain NCIB 11585 are described in our co-pending European Patent Application No. 81300538 (EP—A—35831). Two advantages of strain NCIB 11585 are (a) the urease and GDH enzymes are readily co-purified and (b) the GDH enzyme is constitutive and the alternative pathway using glutamine synthetase and glutamine glutamate aminotransferase cannot occur.

Note:
NCIB is the National Collection of Industrial bacteria, Torrey Research Station, Aberdeen, Scotland, UK;
NRRL is the collection maintained by the US Department of Agriculture, Peoria, Illinois;
FRI is the Fermentation Research Institute, Japan.

When the source micro-organism is a strain of *Methylophilus methylotrophus* such as NCIB 11585 it is suitably produced by a process such as that described in our UK Patent No. 1370892. In such a process a culture of the micro-organism is produced in a fermenter. The method of the invention for the purification together of the enzymes urease and GDH and the production of a micro-organism containing these enzymes may be performed separately, for instance at different locations. Moreover the production of an enzyme component and its admixture with the other components of an assay mixture may be performed

2

together as a two-stage process at the same location or separately at different locations. The extent to which the assay mixture is made up ready for use before being supplied to the user can vary considerably. For instance the enzyme component alone could be supplied, the user then adding everything else. Alternatively the user could be supplied with a mixture fully made up and containing enzymes, cofactors and such other ingredients as stabilisers, which are desirably included, in a buffered medium, i.e. a liquid mixture. Preferably however the user is supplied with an assay mixture which contains the dry ingredients—enzymes, cofactors, buffering materials and other desired ingredients and which on addition to a solvent, usually water, will give the mixture in a suitable buffered medium. The cofactors to be included in the assay mixture are suitably α-keto glutarate and NAD(P)H. Suitable buffering materials include tris(hydroxymethyl) amino methane, triethanolamine, phosphate, imidazole, N-2 hydroxyethylpiperazine-N-2 ethane sulphonic acid (Hepes). Other ingredients include stabilisers, for example albumin (from bovine serum), dithiothreitol or mercapto ethanol.

The amounts of the various components of the mixture in typical assay mixtures are in the following ranges:—

| | |
|---|---|
| urease | 0.5 to 10 units/ml |
| GDH | 2 to 40 units/ml |
| α-ketoglutarate | 0.2 to 10 mM |
| NAD(P)H | 0.05 to 0.5 mM |
| Hepes buffer | 10 to 100 mM—giving a pH in the range 7—8. |

Such a mixture is suitably provided as dried mixture to which water is added to give the concentration indicated.

A preferred mixture has the following composition: urease—1.6 units/ml; GDH—10 units/ml; α-ketoglutarate—1.2 mM; NAD(P)H—0.18 mM and Hepes buffer 50 mM to pH 7.5.

In performing the method of testing using the assay mixture a suitable buffered medium containing it is prepared and a fluid sample e.g. a sample of blood serium is added to it in a conventional manner. Preferably 0.5 to 5 µl of the sample (or equivalent larger volume of diluted material) are added to 0.5 to 5 ml of buffered medium. Blood serum which will be a common material to be tested is formed by removing cells from blood. After addition of the sample e.g. of serum to the buffered medium spectrophotometric measurements are made at 340 nm and the urea content of the test sample is determined. Urea in other fluids such as urine or plasma may be similarly determined.

The source micro-organism for the enzyme component of the assay mixture is preferably grown continuously under carbon limitation and preferably with urea as a nitrogen source. In ideal circumstances cultivation under nitrogen limitation is preferred but we have found that with NCIB 11585 at least this results in some loss of GDH activity. Hence for practical purposes at present growth under carbon limitation is preferred.

In the production of the enzymes from the source, micro-organism cells, suspended in water or buffer, are suitably broken, e.g. mechanically using a bead mill, pressure cell ur ultrasonics, and the material thus produced centrifuged to remove insoluble matter and to give a soluble fraction containing urease and GDH enzymes. This soluble fraction is treated according to steps (A) and (B) of the purification method of the invention. Preferably in step (A) ammonium sulphate is added to the solution of enzymes at 4°C to give 30% saturation with ammonium sulphate. This causes a precipitate of protein not containing the urease and GDH enzymes to form. This precipitate is removed by centrifugation. The ammonium sulphate concentration in the solution is then preferably raised to 50% which causes precipitation of both urease and GDH. The precipitate containing urease and GDH can be re-dissolved, de-salted, e.g. by dialysis, diafiltration or gel chromatography, to remove ammonium sulphate, and treated according to step (B) of the purification method. Preferably in step (B) the enzyme-containing solution in a suitable buffer is applied to a column containing an ion-exchange material, e.g. a column containing a cellulose or other polymer such as diethyl amino ethyl-Sepharose (Sepharose is a trade name for a product of Pharmacia Chemical Co). The enzymes are retained on the column which is then washed with buffer containing an increasing concentration of sodium chloride solution. The urease and GDH enzymes are eluted from the column in the same or closely adjacent fractions at a concentration of sodium chloride between 0.25 and 0.4 M and are collected together.

The purification method of the invention provides a simple and effective means of purifying the urease and GDH enzymes.

The invention is illustrated by the following Example:—

Example

*Methylophilus methylotrophus* strain NCIB 11585 was grown continuously under carbon limitation with urea as nitrogen source. Cells obtained from the culture were broken mechanically and the material

3

**0 069 488**

thus produced was centrifuged to remove insoluble matter and to give a crude extract containing urease and GDH enzymes. The crude extract was dissolved in water and ammonium sulphate was added at 4°C to give 30% saturation with ammonium sulphate. This caused precipitation of protein not containing urease and GDH enzymes. After centrifuging to remove this precipitate further ammonium sulphate was added to raise the ammonium sulphate concentration to 50% and to cause precipitation of both urease and GDH. The precipitate containing urease and GDH was re-dissolved, de-salted and applied to a column containing diethyl amino ethyl (DEAE) Sepharose in 10 mM phosphate buffer pH 7.5. The enzymes were retained upon the column which was washed with an increasing concentration of sodium chloride (0—0.5 M) in the same buffer and the enzymes eluted between 0.25 and 0.4 M, the eluted fractions containing the two enzymes being collected together. The specific activities of urease and GDH in the material at various stages of this purification, i.e. crude extract, precipitate after 30—50% ammonium sulphate treatment and after elution from DEAE Sepharose, are given in Table 1 together with the specific activities of the enzymes in a commercial reagent. In Table 1 specific activity is measured in μ mol/minute/mg protein.

TABLE 1

| Material | Urease specific activity | GDH specific activity |
|---|---|---|
| Crude extract. | 0.25 | 0.45 |
| Precipitate after $(NH_4)_2SO_4$ treatment. | 0.96 | 1.99 |
| Product from DEAE treatment | 4.97 | 14.9 |
| Commercial reagent | 3.21 | 3.86 |

A series of standard urea solutions containing respectively 10, 20, 30, 40 and 50 mg urea per 100 ml solution were made up. The concentrations of urea in these test solutions were then measured using in turn an assay mixture containing the enzyme mixture purified as described above and a commercially available assay mixture. The procedure was to put the assay mixture into a recording spectrophotometer and record absorbance at 340 nm against time obtaining a constant reading. The standard urea solution under test was then added and the reduction in absorbance, due to oxidation of NAD(P)H to NAD(P) as the reaction proceeds, was observed. This takes 2 to 5 minutes. The total change in absorbance is proportional to the amount of urea in the standard solution under test and hence the concentration of urea in the sample can be calculated by conventional means. This was done for each of the standard urea solutions using both the assay mixture of the invention and a commercially available assay mixture. The results are given in Table 2.

The assay mixture comprised 200 μl of an enzyme extract prepared as described above and containing 194 μg protein, 1.2 mM α-ketoglutarate, 0.18 mM NAD(P)H and 50 mM Hepes buffer, being buffered to pH 7.5.

TABLE 2

| Conc. urea added Mg/100 ml | Commercial reagent (1.5 mg protein) | | Assay mixture of invention | |
|---|---|---|---|---|
| | Concentration of urea indicated in assay | Response time | Concentration of urea indicated by assay | Response time |
| 10 | 9.7 mg/100 ml | 2.0 min | 10.1 mg/100 ml | 3.0 min |
| 20 | 21 mg/100 ml | 3.0 min | 21 mg/100 ml | 3.0 min |
| 30 | 32 mg/100 ml | 3.5 min | 32 mg/100 ml | 3.5 min |
| 40 | 41 mg/100 ml | 3.5 min | 41 mg/100 ml | 4.0 min |
| 50 | 52 mg/100 ml | 4.0 min | 51 mg/100 ml | 4.0 min |

As can be seen from Table 2, the results obtained using the assay mixture are comparable with those obtained using an available commercial reagent.

4

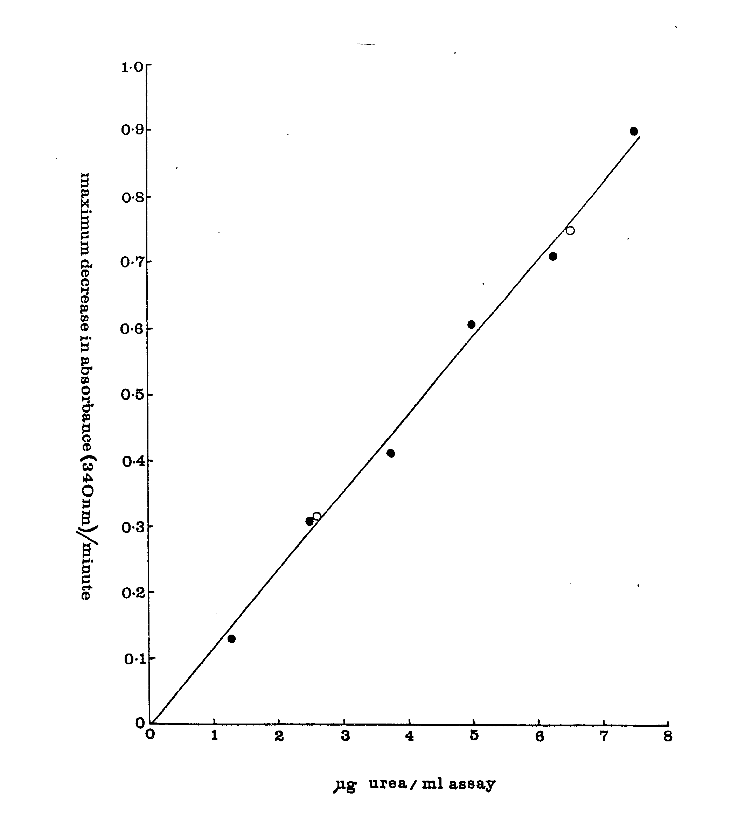

Alternatively the concentration of urea may be calculated from the maximum rate of the reaction which is achieved 5—10 seconds after adding the urea-containing sample to the reaction mixture described above. The concentration of urea in an unknown sample is determined by reference to a curve constructed from reaction rates obtained with standard urea solutions. Such a standard curve and the results of tests with standard serum samples are shown in the Figure. The reaction rate is proportional to the mass of urea in the range 0—8 µg urea/ml assay volume. The results obtained with standard serum samples were in good agreement with the standard curve. This method has the advantage that, since the maximum reaction rate is achieved only 5—10 seconds after mixing, the measurement may be completed in 30 seconds or less so that the procedure is particularly suitable for use with centrifugal reaction rate analysers.

In the Figure the different types of points are shown as follows:—

●: points obtained using urea standards in distilled water;
○: points obtained using standard sera produced by the Wellcome Foundation.

**Claims**

1. A method for the purification together of the enzymes urease and glutamate dehydrogenase (GDH) from the same microorganism source which comprises a step (A) in which a solution containing the enzymes is contacted with ammonium sulphate under conditions such that a precipitate containing both enzymes is produced followed by a step (B) in which the precipitate is redissolved, the resulting solution is applied to an ion exchange material, and a liquid fraction containing the enzymes is collected.

2. A method according to claim 1 wherein in step (A) ammonium sulphate is added to the enzyme-containing solution to cause a first precipitate of protein not containing the enzyme to form, the first precipitate is removed and thereafter the ammonium sulphate concentration in the solution is raised to cause a second precipitate of protein containing urease and GDH enzymes to form.

3. A method according to claim 1 or claim 2 wherein in step (B) the ion exchange matrial is a dextran.

4. A method according to any one of the preceding claims wherein the micro-organism source is a strain of *Methylophilus methylotrophus* which has been genetically modified to include in it the GDH enzyme.

5. A method according to claim 4 wherein the micro-organism source is a strain of *Methylophilus methylotrophus* selected from the Group NCIB 10508—10515 and 10592—10596 which has been genetically modified to include in it the GDH enzyme.

6. A method according to claim 5 wherein the micro-organism source is *Methylophilus methylotrophus* strain NCIB 11585.

**Patentansprüche**

1. Verfahren zur gemeinsamen Reinigung der Enzyme Urease und Glutamatdehydrogenase (GDH) aus demselben als Quelle dienenden Mikroorganismus mit einem Schritt (A), in dem eine Lösung, die die Enzyme enthält, unter derartigen Bedingungen mit Ammoniumsulfat in Berührung gebracht wird, daß ein Niederschlag, der beide Enzyme enthält, erzeugt wird, auf den ein Schritt (B) folgt, in dem der Niederschlag wieder gelöst wird, die erhaltene Lösung auf einen Ionenaustauscher aufgebracht wird und eine flüssige Fraktion, die die Enzyme enthält, gesammelt wird.

2. Verfahren nach Anspruch 1, bei dem in Schritt (A) zu der enzymhaltigen Lösung Ammoniumsulfat hinzugegeben wird, um die Bildung eines ersten Niederschlages aus Protein, das die Enzyme nicht enthält, zu bewirken, der erste Niederschlag entfernt wird und danach die Ammoniumsulfatkonzentration in der Lösung erhöht, wird, um die Bildung eines zweiten Niederschlages aus Protein, das die Enzyme Urease und GDH enthält, zu bewirken.

3. Verfahren nach Anspruch 1 oder 2, bei dem in Schritt (B) der Ionenaustauscher ein Dextran ist.

4. Verfahren nach einem der vorhergehenden Ansprüche, bei dem der als Quelle dienende Mikroorganismus ein Stamm von *Methylophilus methylotrophus* ist, der genetisch modifiziert worden ist, um das Enzym GDH in den Stamm einzubeziehen.

5. Verfahren nach Anspruch 4, bei dem der als Quelle dienende Mikroorganismus ein aus der Gruppe NCIB 10508—10515 und 10592—10596 ausgewählter Stamm von *Methylophilus methylotrophus* ist, der genetisch modifiziert worden ist, um das Enzym GDH in den Stamm einzubeziehen.

6. Verfahren nach Anspruch 5, bei dem der als Quelle dienende Mikroorganismus der Stamm NCIB 11585 von *Methylophilus methylotrophus* ist.

**Revendications**

1. Méthode pour purifier ensemble l'uréase et la glutamate deshydrogénase (GDH) provenant de la même source de microorganisme, qui comprend une étape (A) dans laquelle une solution contenant les enzymes est mise en contact avec du sulfate d'ammonium dans des conditions telles qu'il se forme un précipité contenant les deux enzymes, suivie d'une étape (B) dans laquelle le précipité est redissous, la solution résultante est appliquée à une matière échangeuse d'ion et une fraction liquide contenant les enzymes est collectée.

2. Méthode suivant la revendication 1, caractérisée en ce que dans l'étape (A), le sulfate d'ammonium est ajoutée à la solution contenant les enzymes pour provoquer la formation d'un premier précipité de protéine ne contenant pas les enzymes, le premier précipité est enlevé et ensuite, la concentration en sulfate d'ammonium dans la solution est élevée pour provoquer la formation d'un second précipité de protéine contenant l'uréase et la GDH.

3. Méthode suivant la revendication 1 ou 2, caractérisée en ce que dans l'étape (B), la matière échangeuse d'ion est un dextrane.

4. Méthode suivant l'une quelconque des revendications 1 à 3, caractérisée en ce que la source de microorganisme est une souche de *Methylophilus methylotrophus* qui a été génétiquement modifiée de telle sorte qu'elle inclut la GDH.

5. Méthode suivant la revendication 4, caractérisée en ce que la source de microorganisme est une souche de *Methylophilus methylotrophus* choisie dans le groupe NCIB 10508—10515 et 10592—10596 qui a été modifiée génétiquement de telle sorte qu'elle inclut la GDH.

6. Méthode suivant la revendication 5, caractérisée en ce que la source de microorganisme est la souche NCIB 11585 de *Methylophilus methylotrophus*.